# EUROPÄISCHE PATENTSCHRIFT

(11) **EP 2 987 150 B1**
(45) Veröffentlichungstag und Bekanntmachung des Hinweises auf die Patenterteilung: **03.06.2020**
(21) Anmeldenummer: 14716543.5
(22) Anmeldetag: 04.04.2014
(51) Int. Cl.: G08B 3/10, A47K 5/12, G08B 21/24

(54) **TRAGBARE VORRICHTUNG ZUR VERBESSERUNG DER HYGIENE SOWIE VERFAHREN**
PORTABLE DEVICE FOR IMPROVING HYGIENE AND METHOD
DISPOSITIF PORTABLE D'AMÉLIORATION DE L'HYGIÈNE, ET PROCÉDÉ

(30) Priorität: 16.04.2013 DE 102013006494
(43) Veröffentlichungstag der Anmeldung: 24.02.2016
(73) Patentinhaber: GWA Hygiene GmbH, 18435 Stralsund (DE)
(72) Erfinder: HERZOG, Andreas, 60438 Frankfurt (DE)
(74) Vertreter: Röthinger, Rainer
(86) Internationale Anmeldenummer: PCT/EP2014/056837
(87) Internationale Veröffentlichungsnummer: WO 2014/170145

(56) Entgegenhaltungen:
- WO-A1-02/091297
- JP-A- 2009 223 775
- US-A1- 2010 073 162

## Beschreibung

Die vorliegende Erfindung betrifft eine tragbare Vorrichtung zur Verbesserung der Hygiene sowie ein Verfahren.

### Grundlagen

Eine geeignete Handhygiene ist in vielen Branchen von Bedeutung, u.a. in Krankenhäusern und Pflegeheimen zur Vermeidung nosokomialer Infektionen, in der Nahrungsmittelindustrie und Industrie zur Vermeidung von Kontaminationen, sowie allgemein überall dort, wo die Übertragungskette für Krankheitserreger und Kontaminationen bei dem typischen Überträger "Hand" unterbrochen werden soll. Besonders bedeutsam ist die Handhygiene in medizinischen Einrichtungen.

Die Einhaltung der Handhygieneregeln durch das Personal und Besucher von Einrichtungen ist häufig nicht optimal, sinnvolle oder vorgeschriebene Handhygienemaßnahmen unterbleiben, z.B. da sie zum vorgeschriebenen Zeitpunkt vergessen werden.

Zur Verbesserung der Einhaltung werden folgende grundlegende Ansätze verwendet:
1. Rechtzeitige Erinnerung an die Handhygiene, um eine Handhygienemaßnahme anzustoßen, sowie Detektion einer regelgemäß notwendigen Handhygienemaßnahme. Diese Maßnahme bezieht sich auf den Sollwert von Hygienemaßnahmen.
2. Quantitative Erfassung von Handhygienemaßnahmen oder geeigneter Surrogatparameter, um Erkenntnisse zu gewinnen, die in weitere Maßnahmen (z.B. Training) zur Verbesserung des Handhygieneverhaltens münden. Dieser Ansatz bezieht sich auf den Istwert von Hygienemaßnahmen.
3. Steigerung der Transparenz des individuellen Handhygieneverhaltens, um eine Selbstkontrolle, Kontrolle oder Erinnerung durch Dritte zu ermöglichen. Diese Maßnahme bezieht sich auf das Verhältnis von Istwert und Sollwert von Hygienemaßnahmen.

### Stand der Technik

Stand der Technik für die Erinnerung an die Handhygiene (Ansatz 1) sowie die Detektion einer notwendigen Handhygienemaßnahme sind in erster Linie zonenbasierte Verfahren und zeitbasierte Verfahren. Bei zonenbasierten Verfahren wird das Eintreten in oder Verlassen von vorab definierten Zonen mit geeigneter Technik (z.B. Funktechnologien, optische Erfassung) überwacht und nach bestimmten Algorithmen ein Signal an das Personal gegeben bzw. eine Auswertung erzeugt. Zeitbasierte Verfahren erinnern in bestimmten Zeitabständen an die Handhygiene, die sich nur eher zufällig mit den Indikationen zur Handhygiene decken.

Ein Nachteil der zonenbasierten Verfahren besteht darin, dass hierfür spezielle Zonen ex ante, d.h. vor der Anwendung der Systeme oder Geräte für die Erinnerung an die Handhygiene, definiert werden müssen und mit typischerweise ortsgebundener Technologie (z.B. RfID-Sender) überwacht werden müssen. Dafür sind kostenintensive lokale Installationsarbeiten erforderlich. Darüber hinaus ist die Flexibilität eingeschränkt. Im Anwendungsfall eines Krankenhauses werden z.B. häufig Betten in Räumen umgestellt, so dass die betroffenen Zonen jedes Mal neu eingerichtet werden müssen.

Ein weiterer Nachteil der zonenbasierten Verfahren besteht in datenschutz-, personal- und haftungsrechtlichen Aspekten, da nachvollziehbar wird, wo genau eine Person sich zu einer bestimmten Zeit aufgehalten hat. Darüber hinaus können funkbasierte Verfahren zur Zonenüberwachung Akzeptanzschwierigkeiten hervorrufen, insbesondere wenn Strahlungen oder Interferenzen mit anderen, wichtigen Funksystemen oder elektronischen Geräten im jeweiligen organisatorischen Umfeld subjektiv befürchtet werden.

In Bezug auf die Erfassung von durchgeführten Handhygienemaßnahme (Ansatz 2) ist Stand der Technik die Erfassung des Desinfektionsmittelverbrauchs als Surrogatparameter, oder alternativ die Erfassung der Anzahl von Betätigungen von stationären Desinfektionsmittelspendern. Diese Ansätze können nur eine sehr ungenaue Erfassung der tatsächlichen Häufigkeit von Handhygienemaßnahmen erreichen. Bei Verbrauchsmessungen ist nicht klar, ob der Verbrauch durch erfolgreiche Handhygiene oder z.B. durch Diebstahl oder Verschwendung von Handhygienemittel erfolgte. Auch die Messung der Anzahl der Betätigungen von Desinfektionsmittelspendern gibt nur eingeschränkt Auskunft, ob tatsächlich eine Handhygienemaßnahme mit der vorgeschriebenen Einwirkungsdauer der verwendeten Substanz (im Falle von Desinfektionsmitteln z.B. mindestens 30 Sekunden nach WHO-Regeln) erfolgte. Insbesondere ist aber eine Erfassung von Desinfektionen mit gemäß Studien äußerst desinfektionshäufigkeitssteigernden zusätzlichen, nicht-stationären Spendern von Desinfektionsmitteln, insbesondere Kittelflaschen, kaum praktikabel, da die Erfassung auf diese Weise eine Ausstattung der Spendersysteme mit geeigneter Technologie voraussetzt. Untersuchungen haben aber gezeigt, dass die ubiquitäre Verfügbarkeit von Handhygienemitteln eine wesentliche Steigerung des Verbrauchs und somit Verbesserung des Handhygieneverhaltens nach sich zieht. Diese ubiquitäre Verfügbarkeit ist mit aufwändigen, technologisch aufgerüsteten stationären Spendern nicht zu erreichen.

In Bezug auf die Steigerung der Transparenz des individuellen Handhygieneverhaltens (Ansatz 3), bei der Istwert und Sollwert der Handhygienemaßnahmen miteinander in Beziehung gesetzt werden, ist der Stand der Technik dadurch gekennzeichnet, dass unterschiedliche Erfassungssysteme und -vorrichtungen an verschiedenen Orten die Handhygienemaßnahmen sowie die Notwendigkeit von Handhygienemaßnahmen detektieren. Diese Informationen werden im Stand der Technik an zentraler Stelle - typischerweise durch Funkverbindungen - zusammengetragen, ausgewertet und an einen Signalgeber, typischerweise an der hygienepflichtigen Person befestigt, weitergegeben. Die funkgestützte Kommunikation kann in bestimmten Organisationsumgebungen aufgrund von nicht ausschließbaren elektromagnetischen Störungen empfindlicher Geräte oder aufgrund subjektiver Befindlichkeiten von Nutzern nachteilig sein. Das System wird durch die mehreren beteiligten Komponenten insgesamt komplex und somit aufwändig und störungsanfällig.

Patent DE 10 2011009 240 A1 beschreibt ein System zur Hygienekontrolle. Es basiert im Wesentlichen auf einem Näherungssensor und erinnert den Benutzer mittels Signalton an die Benutzung des Reinigungsmittels. Es wird auch vorgeschlagen, dass nach einem definierten Zeitintervall nach Annäherung und Entfernung einer Person an den Sensor der Warnton ausgegeben wird. Zur Markierung der eintretenden Personen wird ein RfID-Tracking vorgeschlagen. Nachteilig ist bei dieser Lösung unter anderem, dass alle Hygienebereiche in ihrer Ausdehnung ex ante vollständig fest definiert werden müssen und somit die operative Flexibilität etwa im Klinikbetrieb einschränken, dass fest installierte Näherungssensoren vorhanden sein müssen, und dass das System als Desinfektionsmittelspender nicht den hygieneförderlichen Einsatz von mobilen Kittelflaschen und zusätzlichen mobilen Spendern vorsieht.

Auch das Patent DE 10 2012 105 368 A1 beschreibt ein System zur Hygienekontrolle. Hier wird ein Sensor und/oder bei Reinigungsstation/Waschbecken o.ä. in Kombination mit einem tragbaren Identifikationsmittel vorgeschlagen, um die Einhaltung von Hygienevorschriften zu überwachen. Die Überwachung erfolgt über einen kapazitiven Sensor, der reagiert, wenn der Benutzer seine Hände mit Wasser/Desinfektionslösung benetzt. Die Kommunikation zwischen Sender und Empfänger erfolgt drahtlos. Es wird vorgeschlagen, einen Warnton zu senden, wenn der Benutzer entweder eine bestimmt Zeit keine Reinigungsstation aufgesucht hat, oder wenn er einen bestimmten Raumabschnitt betreten bzw. verlassen hat. Diese Lösung ist mit einer Raumüberwachung verbunden, so dass diese aus datenschutzrechtlichen Gründen und Akzeptanzgründen problematisch ist. Ferner wird eine Vielzahl von Sensoren zur Überwachung der Räume benötigt.

Patent Nr. US 2010/0073162 beschreibt ein Gerät zur Erinnerung an die Handhygiene. Zur Erkennung eines Handhygienevorgangs wird die Kombination aus einem Bewegungssensor, Gassensor und ggf. akustischem Sensor vorgeschlagen. Der Bewegungssensor wird nicht zur Schrittzählung verwendet, sondern lediglich, um festzustellen, ob Objekte berührt wurden. Da bei arbeitsplatztypischen Verrichtungen jedoch eine Vielzahl von Objekten in schneller Folge nacheinander berührt werden, ohne dass jedes Mal eine Indikation zur Handhygiene gegeben ist, führt diese Art der Bewegungsmessung in erheblichem Maße zu Fehlinformationen über das Vorliegen der Indikation für eine Handhygienemaßnahme. Darüber hinaus erfordert diese Erfindung das Tragen eines Gerätes am Handgelenk bzw. in der Nähe der Hand, um Berührungen erkennen zu kennen. Dies ist nach geltenden Hygienevorschriften vieler Einrichtungen, z.B. Krankenhäuser, aus hygienischen Gründen unzulässig. Schließlich basiert die Erfindung auf der Verwendung mehrerer räumlich getrennter Sensoren und Signalgeber, was Datenübertragung zwischen den jeweiligen Gehäusen erforderlich macht und die Praktikabilität einschränkt.

Das Patent JP 20010-010982 A beschreibt eine Methode zur Verteilung der Arbeitsbelastung von Pflegekräften. Hierbei wird ein Schrittsensor zur Feststellung der Laufleistung verwendet. Es ist Stand der Technik, dass man mit Pedometern eine Laufleistung oder sogar auch die Länge einer zurückgelegten Wegstrecke erfassen kann. Eine Laufleistungsmessung ist aus der üblichen Verwendung von Pedometern naheliegend.

Bei der vorliegenden Erfindung geht es dagegen nicht um eine Laufleistungsmessung oder Arbeitslastverteilung wie im zitierten Patent, sondern um die Feststellung eines speziellen Ereignisses, festgemacht an einem Ortswechsel. Dies ist ein ganz anderer, neuartiger und vor dem Hintergrund der üblichen Verwendung eines Pedometers überraschender Verwendungszweck.

Patent US 2012/0055986 A1 beschreibt ein System zur Verbesserung der Hygiene in medizinischen Einrichtungen, wobei das System eine tragbare Datenleseeinheit umfasst, die von einer Türauslöseeinheit erkannt werden kann, falls eine mit der Datenleseeinheit ausgestattete Person einen Raum betritt, und eine Spenderauslöseeinheit, die eine Hygienebehandlung einer mit der Datenleseeinheit ausgestattete Person erkennen kann. In einem für die Lehre dieser Druckschrift unwesentlichen Absatz wird dargelegt, dass die tragbare Datenleseeinheit eine Auswertung des Hygieneverhaltens eines Trägers erlaubt, wobei eine Analogie zu einem Pedometer dargestellt wird. Der Analogiegegenstand ist allein die angestrebte Verhaltensänderung des Menschen. Die Erwähnung des Pedometers hat nichts mit der erfindungsgemäßen Verwendung des Pedometers für die Erkennung eines hygienerelevanten Ortswechsels zu tun. Die tatsächliche Verwendung eines Pedometers ist sogar explizit nicht Gegenstand des dort angemeldeten Patents, es ist lediglich im Sinne einer Metapher erwähnt. Im Gegenteil zeigt diese Erwähnung eines Pedometers im Zusammenhang mit einem anderen Patent, das der Verbesserung der Handhygiene dient, ohne dass eine erfindungsgemäße Ausgestaltung und Verknüpfung eines Pedometers erfolgt, sehr eindringlich, dass diese eines Pedometers zur Detektion eines Ortswechsels eben nicht naheliegend ist.

Das Dokument WO 02/091297 erwähnt zwar indirekt einen Schrittzähler, seine Distanz-Signale werden aber an eine zentrale Hauptstation geschickt. Eine Warnsignalisierung an der tragbaren Vorrichtung findet nicht statt, es wird nur eine ortsbasierte Überwachung mit anderen festen Sensoren im Gebäude getätigt.

### Gegenstand der Erfindung

In Anbetracht des Standes der Technik ist es daher Aufgabe der vorliegenden Erfindung, die durch die Ansprüche definiert ist, ein technisch verbessertes System zur Verbesserung der Hygiene, insbesondere in medizinischen Einrichtungen bereitzustellen, das nicht mit den Nachteilen herkömmlicher Verfahren behaftet ist.

Insbesondere sollte Personal rechtzeitig an Handhygienemaßnahmen erinnert werden, wie sie gemäß den Handhygieneregeln der WHO insbesondere auch vor und nach Patientenkontakt und nach Verlassen der Patientenumgebung geboten sind. Hierbei sollte es möglich sein, aus datenschutzrechtlichen Gründen oder Akzeptanzgründen auf eine Erhebung von individualisierten Daten zu verzichten. Weiterhin sollte das System möglichst einfach und kostengünstig ausgestaltet werden können.

Gelöst werden diese sowie weitere nicht explizit genannte Aufgaben, die jedoch aus den hierin einleitend diskutierten Zusammenhängen ohne weiteres ableitbar oder erschließbar sind, durch ein tragbare Vorrichtung zur Verbesserung der Hygiene mit allen Merkmalen des Patentanspruchs 1.

Gegenstand der vorliegenden Erfindung ist dementsprechend eine tragbare Vorrichtung zur Verbesserung der Hygiene, umfassend mindestens eine Warneinheit, die mit einer Einheit zur Detektion einer Desinfektionsbehandlung derart zusammenwirkt, dass ein Signal auslösbar ist, welche dadurch gekennzeichnet ist, dass die Warneinheit mit mindestens einer Schrittzähleinheit in Wirkverbindung steht.

Die Erfindung vermeidet die Nachteile, insbesondere in Bezug auf den zuvor näher erläuterten Ansatz 1, der Erinnerungsfunktion, durch einen grundsätzlich ortsungebundenen Ansatz, der gleichwohl die Indikationen zur Handhygiene erfasst, die auf einer Veränderung des Aufenthaltsortes des Hygienepflichtigen basieren.

In einer besonderen Ausprägung setzt die Erfindung die typischerweise in einer Organisation bestehenden Handhygieneregeln um. Weltweit im Einsatz und Grundlage für zahlreiche nationale und organisationsspezifische Handhygieneregelungen sind die Handhygieneregeln der WHO, nach denen u.a. vor Patientenkontakt, nach Patientenkontakt, sowie nach Kontakt mit einer Patientenumgebung eine Handhygienemaßnahme erforderlich ist, d.h. in anderer Terminologie eine Indikation zur Handhygiene gegeben ist.

Der vorliegenden Erfindung liegt die Erkenntnis zu Grunde, dass diese Indikationen mit einer Ortsveränderung des Hygienepflichtigen verbunden sind. Überraschend können die zuvor dargelegten Ziele einer Verbesserung der Hygiene insbesondere auch in medizinischen Einrichtungen dadurch erzielt werden, dass durch spezifische Schrittbewegungen erfolgte Ortsveränderungen mit einer Erinnerung an eine Hygienemaßnahme gekoppelt werden. Eine Ortsveränderung geht typischerweise mit einer bestimmten Mindestanzahl von Schritten einher. Durch die Erfassung dieser Schrittzahl ist somit typischerweise die Erfassung dieser Indikationen möglich. Umgekehrt ist ein durch eine gewisse Mindestschrittzahl gekennzeichneter Ortswechsel im Umfeld insbesondere eines Krankenhauses typischerweise mit einer Indikation zur Handhygiene verbunden, also z.B. auch der Toilettengang, der Gang aus Pausenräumen in Patientenzimmer, etc. Die Erfassung der Überschreitung einer gewissen Mindestschrittzahl ist somit ein guter Indikator für die Indikation zur Handhygiene.

Durch die vorliegende Erfindung ist es möglich, Hygienepflichtige auch nach einem Patientenkontakt und nach dem Verlassen einer Patientenumgebung an eine Hygienemaßnahme zu erinnern, ohne dass eine individualisierbare Raumüberwachung stattfinden muss. Aufwändig fest installierte Überwachungssysteme und Kommunikation zwischen fest installierten und mobilen Einheiten eines Überwachungssystems werden überflüssig.

Erfindungsgemäß ist insbesondere auch eine Ausprägung, in der die Messung der Schrittzahl mit weiteren Indikatoren für das Vorliegen einer Handhygieneindikation kombiniert wird, z.B. Empfang eines Infrarotsignals in bestimmten besonders überwachten Zonen, Erfassung von bestimmten Bewegungsmustern, zeitlichen Abständen, etc.

In Bezug auf Ansatz 2 - Erfassung von Hygienemaßnahmen - ermöglicht die Erfindung eine wesentlich präzisere Erfassung tatsächlich erfolgter Handhygienemaßnahmen, indem die Anwesenheit von Handhygienemittel in hoher Konzentration in unmittelbarer Handnähe - und somit mit hoher Wahrscheinlichkeit von einer Handhygienemaßnahme herrührend - durch die Vorrichtung detektiert wird, und zudem die Einhaltung der erforderlichen Einwirkungszeit des Handhygienemittels durch mehrfache Messung der Gaskonzentration überwacht wird. Die besonders wichtige Verwendung von möglichst überall verfügbaren dezentralen Handhygienemittelspendern wird durch die erfindungsgemäße Erfassung von Handhygieneereignissen durch eine mobile, am Träger befestigte Vorrichtung im Gegensatz zum Stand der Technik bei der Erfassung nicht eine Erhöhung der Spenderkosten eingeschränkt. Eine reine Messung des Verbrauchs des Handhygienemittels macht im Vergleich dazu keine Aussage über dessen Verwendung, die z.B. auch Diebstahl oder Verschwendung zur Schönung der Verbrauchsstatistik beinhalten könnte.

In der besonderen Ausprägung der Erfindung bei Einsatz in Krankenhäusern sind die verwendeten Gassensoren Alkoholsensoren, da die in Krankenhäusern eingesetzten Desinfektionsmittel typischerweise auf der Basis eines erheblichen Alkoholgehalts wirken.

Eine erfindungsgemäße tragbare Vorrichtung zur Verbesserung der Hygiene umfasst mindestens eine Warneinheit, die mit einer Einheit zur Detektion einer Desinfektionsbehandlung derart zusammenwirkt, dass ein Signal auslösbar ist. Eine Warneinheit bezeichnet hierbei eine Einheit, die ein Signal auf Basis einer detektierten Desinfektionsbehandlung steuern kann. Im Allgemeinen können hierzu bekannte Datenverarbeitungseinheiten oder Steuerungseinrichtungen, wie Mikrocontroller eingesetzt werden. Die in der Warneinheit im Allgemeinen vorgesehene Steuerungseinrichtung wertet die entsprechenden Signaldaten der Einheit zur Detektion einer Desinfektionsbehandlung und der Schrittzähleinheit mit geeigneten Algorithmen aus. In Abhängigkeit der Auswertung kann die Warneinheit Signale auslösen. Diese Signale können unter anderem visuell, beispielsweise durch einen LCD-Bildschirm, der berührungsempfindlich ausgestaltet werden kann, oder eine LED-Leuchte, akustisch, beispielsweise durch Lautsprecher oder über Bewegungen, vorzugsweise Vibrationen dem Hygienepflichtigen mitgeteilt werden.

So kann die Warneinheit beispielsweise eine Kontrollanzeige zur Anzeige des Desinfektionszustandes, beispielsweise eine oder mehrere LEDs, einen Lautsprecher oder eine Einheit zur Erzeugung eines Vibrationsalarms zur Erinnerung an die Desinfektion umfassen.

Die Detektion eines Handhygienevorgangs kann durch ein geeignetes Signal, z.B. eine grün leuchtende LED, angezeigt werden. In einer besonderen Ausprägung ist dieses Signal nur für den Träger der erfindungsgemäßen Vorrichtung, oder in einer anderen Ausprägung zur Erleichterung einer sozialen Kontrolle zusätzlich für Personen in der Nähe des Trägers der erfindungsgemäßen Vorrichtung wahrnehmbar.

Bei der Feststellung einer Indikation zur Handhygiene nach Auswertung der Signale der Einheit zur Detektion einer Desinfektionsbehandlung durch die Warneinheit kann beispielsweise ein Lichtsignal an der LED gegeben werden, z.B. ein rotes Licht bei Verwendung einer RGB-LED, oder ein bestehendes Lichtsignal, das eine erfolgreiche Handhygiene indiziert, ausgeschaltet werden.

Die LED kann zusätzlich zur Signalisierung weiterer relevanter Betriebszustände der erfindungsgemäßen Vorrichtung, z.B. eine Pausenschaltung oder einen Aufladungszustand, verwendet werden.

Zur Erinnerung an eine erforderliche Handhygienemaßnahme kann ein Alarmgeber eingesetzt werden. In besonderen Ausprägungen können dies ein Vibrationsalarm oder ein Tonsignal sein. Dabei ist erfindungsgemäß zwischen der erforderlichen Aufmerksamkeit für das Signal und der Akzeptanz beim Träger der erfindungsgemäßen Vorrichtung abzuwägen.

In einer weiteren besonderen Ausprägung der Erfindung können weitere I/O-Elemente für Dateneingabe und -ausgabe, z.B. Displays und Touchscreens, weitere Informationen über situationsgerecht indizierte Hygienemaßnahmen, weitere Maßnahmen, Hintergrundinformationen etc. angezeigt werden und Interaktionen mit dem Träger der erfindungsgemäßen Vorrichtung durchgeführt werden.

Einheiten zur Detektion einer Desinfektionsbehandlung sind im zuvor beschriebenen Stand der Technik dargelegt. Beispielsweise können Funkverbindungen oder RfID-Chips eingesetzt werden, die bei einer Näherung an einen Spender ein Signal an die Warneinheit übermitteln, um Desinfektionsbehandlungen festzustellen.

Die Funkverbindungen können unter anderem mit WLan und/oder Bluetooth realisiert werden, alternativ ist auch eine Infrarot-Verbindung möglich.

Weiterhin kann die Einheit zur Detektion einer Desinfektionsbehandlung mit einem Sensor, beispielsweise einem Näherungssensor oder einem kapazitiven Sensor, wie dieser ausführlich in DE 10 2012 105 368 A1 dargestellt ist, ausgestattet sein, der bei einer Betätigung von einem Desinfektionsmittelspender eine Desinfektionsbehandlung feststellt. Ferner kann die Einheit zur Detektion einer Desinfektionsbehandlung mit einem Schalter ausgestattet werden, der eine manuelle Bestätigung einer Desinfektionsbehandlung ermöglicht.

Vorzugsweise umfasst die Einheit zur Detektion einer Desinfektionsbehandlung mindestens einen Gassensor, der vorzugsweise gegenüber Alkohol, besonders bevorzugt Ethanol und/oder Propanol, empfindlich ist. Durch diese Ausgestaltung kann eine Detektion überall mobil und abhängig von einzelnen Spendern erfolgen und gleichzeitig auch die Dauer einer Desinfektionsbehandlung besonders einfach festgestellt werden, so dass hohe Hygienestandards erreicht werden können.

Der Gassensor kann vorzugsweise mittels geeigneter Auswertungssoftware die Gegenwart von für die Handhygiene verwendeten chemischen Substanzen detektieren. Die chemische Substanz kann entweder ein notwendiger Bestandteil des Handhygienemittels sein oder eine zu Detektionszwecken zuvor zugefügte Substanz. Durch mehrfache Messung der Anwesenheit der Substanz im Zeitablauf kann, in einer besonderen Ausprägung, darüber hinaus auf eine erfolgreiche Handhygienemaßnahme, die auch durch eine bestimmte Einwirkungsdauer der Substanz gekennzeichnet ist, geschlossen werden. Darüber hinaus kann durch diese Maßnahme die Wahrscheinlichkeit einer zufälligen Erfassung der Substanz, die in der Nähe nicht für die Handhygiene des Trägers der Vorrichtung verwendet wird, verringert werden.

In einer besonderen Ausprägung der Erfindung wird ein Alkoholsensor als Gassensor verwendet. Da die im Krankenhaus zugelassenen Händedesinfektionsmittel von praktischer Bedeutung auf Alkoholbasis wirken und ein bloßes Händewaschen mit Seife nicht den Regeln für die Handhygiene entspricht, kann dadurch auf eine durchgeführte Händedesinfektion geschlossen werden. Durch geeignete Einstellung des Schwellwertes für die Erkennung (nicht zu hoch, so dass jede Desinfektion erkannt wird, aber auch nicht zu tief, so dass Desinfektionen anderer Personen nicht erkannt werden) sowie durch mehrmalige Wiederholung der Messung im Zeitablauf (zwecks Ausschluss der fälschlichen Messung einer vorbeiziehenden Alkoholwolke z.B. aus der Desinfektion anderer Personen, sowie zusätzlich zwecks Feststellung der erforderlichen Einwirkungsdauer des Desinfektionsmittels) kann eine hohe Präzision bei der Erfassung von Desinfektionsereignissen erzielt werden.

Die Auswertung der über die Einheit zur Detektion einer Desinfektionsbehandlung erfassten Daten kann durch eine separate Datenverarbeitungseinheit oder Steuerungseinrichtung erfolgen. Ferner kann vorgesehen sein, dass diese Auswertung über die zuvor beschriebene Warneinheit erfolgt.

Die Warneinheit steht mit mindestens einer Schrittzähleinheit in Wirkverbindung. Die Schrittzähleinheit umfasst im Allgemeinen einen Beschleunigungssensor, vorzugsweise einen Bewegungssensor über dessen Daten mit geeigneter Software ein Bewegungsmuster erkennbar ist, welches beispielsweise einer vorgegebenen Zahl an unmittelbar hintereinander folgenden Schritten und/oder einer Schrittzahl von zeitlich relativ gleichmäßig aufeinanderfolgenden Schritte entspricht. Daraus können bei geeigneten Schwellwerten für eine Mindestschrittzahl sowie in einer besonderen Ausprägung der Erfindung auch auf Basis eines Algorithmus in Kombination mit anderen Informationen mit hoher Zuverlässigkeit die Indikationen für eine Händedesinfektion vor Patientenkontakt, nach Patientenkontakt und nach Verlassen der Patientenumgebung nach den Regeln der WHO abgeleitet werden.

Die Möglichkeiten zur Operationalisierung der Schritterkennung sind in der Fachwelt weithin bekannt und unter anderem in Ms. Najme Zehra Naqvi et al. / International Journal on Computer Science and Engineering (IJCSE), Vol. 4 No. 05 May 2012, ISSN: 0975-3397, oder in United States Patent Application 20140074431, oder Neil Zhao, Full-Featured Pedometer Design Realized with 3-Axis Digital Accelerometer, Analog Dialogue 44-06, June (2010), beschrieben.

Die zuvor näher beschriebenen Verfahren können jeweils dadurch ergänzt werden, dass aufgrund einer zeitlichen Abfolge von Beschleunigungswerten eine Schrittfolge festgestellt wird, die als unmittelbar hintereinander folgende Schritte und/oder zeitlich relativ gleichmäßig aufeinanderfolgende Schritte klassifiziert werden kann. Wenn also beispielsweise innerhalb eines vorzugebenden zeitlichen Intervalls, innerhalb dessen normalerweise ein Folgeschritt innerhalb einer bestehenden Schrittfolge erfolgt, kein weiterer Schritt detektiert wird, dann wird das Ende der Schrittfolge erkannt. Sofern diese Schrittfolge eine Mindestschrittzahl überschreitet, wird ein hygienerelevanter Ortswechsel erkannt.

Zur Erkennung eines Bewegungsmusters, welches einer vorgegebenen Zahl an unmittelbar hintereinander folgenden Schritten und/oder einer Schrittzahl von zeitlich relativ gleichmäßig aufeinanderfolgenden Schritte entspricht, wird neben einer Beschleunigungsinformation auch eine Zeitinformation verarbeitet, wobei diese Zeitinformation zusammen mit der Beschleunigungsinformation in ein zeitliches Intervall überführt werden kann, innerhalb dessen ein Folgeschritt erfolgen muss, um als zur Schrittfolge zugehörig erkannt zu werden.

Das zeitliche Intervall, innerhalb dessen ein Folgeschritt erfolgen muss, um als zur Schrittfolge zugehörig erkannt zu werden, sowie die anderen zur Erkennung erforderlichen Zeit- und Beschleunigungsschwellwerte, können entweder für alle Träger einer Einrichtung mit erhöhten Hygieneanforderungen softwareseitig vorgegeben werden (z.B. auch anhand der Charakteristika der Organisation, innerhalb derer das System angewandt wird). Diese Werte können aber in einer vorteilhaften Ausprägung auch individuell anhand der Charakteristika der Gangart des jeweiligen Trägers eingegrenzt werden, davon ausgehend, dass Personen unterschiedliche, typische Ganggeschwindigkeiten, Schritt- und Beschleunigungscharakteristika haben. Auf diese Art kann eine höhere Erkennungsgenauigkeit erzielt werden. Dazu können die geeigneten Parameter in einer Lernphase ermittelt werden. Die hierbei gewonnenen Daten können in eine von einem Träger zu verwendende Vorrichtung zur Verbesserung der Hygiene individuell einprogrammiert werden. Weiterhin können für verschiedene Trägergruppen unterschiedliche Klassen von Vorrichtungen zur Verbesserung der Hygiene bereitgestellt werden, so dass besonders große Träger einen Typ tragen, während kleinere Anwender einen anderen Typus einsetzen. Gemäß einer besonderen Ausgestaltung kann eine Vorrichtung zur Verbesserung der Hygiene bei einem Arbeitsbeginn einer Person entsprechend dieser Person programmiert werden, wobei dies vorzugsweise durch Eingabe eines Codes erfolgen kann. Diese Eingabe (Personalisierung) kann an einer Basisstation erfolgen, an der die Vorrichtung aufgeladen werden kann, wie dies später ausführlich dargelegt wird. Ferner kann der Code dem speziellen Gerät auch durch Funktechnik und/oder über eine Infrarot-Schnittstelle mitgeteilt werden.

Insbesondere bei personalisierten oder durch Code-Eingabe personalisierbaren tragbaren Vorrichtungen kann die Lernphase auch einen längeren Zeitraum umfassen, so dass entsprechend engere Grenz- und Schwellwerte möglich sind, und auch im Zeitraum auftretende unterschiedliche Gangarten (z.B. langsames Gehen, schnelles Gehen und Laufen) unterschieden werden können.

Die anhand der Beschleunigungswerte und der zeitlichen Abstände der Schritte mögliche Unterscheidung unterschiedlicher Gangarten (z.B. langsames Gehen, schnelles Gehen, Rennen, entweder individuell ermittelt oder standardmäßig vorgegeben) können auch mit einer Abschätzung der jeweiligen Schrittlänge verbunden werden. Dies gilt in besonderem Maße bei Verwendung von personalisierten tragbaren Vorrichtungen, für die die Schrittlänge des Trägers bei verschiedenen Gangarten eingegeben werden kann, beispielsweise durch Tasteneingaben oder durch Nutzung einer Datenverbindung zur tragbaren Vorrichtung. Individuelle Schrittlängen können in einer weiteren Ausprägung der Erfindung auch statistisch ermittelt werden, indem die Anzahl der Schritte einer bestimmten Gangart zwischen zwei aus der absoluten Positionsbestimmung mit einem Alternativverfahren (z.B. Infrarot, RfID, Bluetooth, GPS) ermittelt wird und die bekannte Wegstrecke zwischen den beiden Absolutpositionen durch die Anzahl der zurückgelegten Schritte geteilt wird.

Gemäß einer besonderen Ausprägung der vorliegenden Erfindung, kann anhand der Schrittlängen innerhalb der ununterbrochenen Schrittfolge auch die zurückgelegte Entfernung innerhalb der Schrittfolge errechnet werden. In diesem Fall kann im Rahmen der Erfindung die Erkennung eines Ortswechsels anhand einer bestimmten Mindestschrittzahl innerhalb einer ununterbrochenen Schrittfolge durch die Erkennung eines Ortswechsels anhand der Entfernungsschätzung auf Basis der Anzahl der Schritte innerhalb einer ununterbrochener Schrittfolge ersetzt werden.

Der Beschleunigungssensor kann, in einer weiteren besonderen Ausprägung, zusätzlich die Aktivität des Trägers erfassen und somit ermöglichen, dass die Anzahl der Handhygienemaßnahmen und der Indikationen für Handhygienemaßnahmen mit der Anzahl der tatsächlichen Personaleinsatzstunden in Bezug gesetzt werden kann, was z.B. für eine statistische Auswertung von Bedeutung sein kann. Er kann ferner weitere, für die Indikationsstellung relevante Bewegungsmuster erkennen.

Die Auswertung der über die Schrittzähleinheit erfassten Daten kann durch eine separate Datenverarbeitungseinheit oder Steuerungseinrichtung erfolgen. Weiterhin kann vorgesehen sein, dass diese Auswertung über die zuvor beschriebene Warneinheit erfolgt.

Ferner kann vorgesehen sein, dass die Warneinheit mit mindestens einer Schrittzähleinheit über eine Datenverbindung derart verbunden ist, dass über die Schrittzähleinheit erhaltene Daten in die Warneinheit übertragbar sind, und die übertragenen Daten in der Warneinheit so verarbeitbar sind, dass ein Signal auslösbar ist.

Darüber hinaus kann die Vorrichtung auch mindestens eine Zeiterfassungseinheit umfassen, die vorzugsweise in Wirkverbindung mit der Warneinheit steht. Darüber können beispielsweise ergänzende, zusätzliche Erinnerungen an den Träger der Vorrichtung gegeben werden.

Ferner kann die erfindungsgemäße Vorrichtung einen IR-Sensor (Infrarot-Sensor)aufweisen, der in Wirkverbindung mit der Warneinheit steht. Dadurch kann in bestimmten Zonen eine zusätzliche Erinnerung des Trägers der Vorrichtung erreicht werden. Ferner kann eine andere Art der Überwachung der Schrittfolge, z.B. mit einem verringerten Schwellwert für die Mindestschrittzahl, nach der die Warneinheit aktiviert wird, erzielt werden. Weiterhin kann auf diese Weise die Möglichkeit zur Interaktion mit dem Nutzer mittels einer Infrarot-Fernbedienung, z.B. zur Eingabe eines persönlichen Codes, geschaffen werden.

In einer besonderen Ausprägung der Erfindung werden Beschleunigungssensor und Gassensor durch einen zusätzlichen Infrarotsensor ergänzt. In ausnahmsweise auftretenden Situationen, in denen relevante räumliche Veränderungen so klein sind, dass sie nicht durch den Beschleunigungssensor in der Verwendung als Schrittsensor detektiert werden können, kann ein zusätzlicher Infrarotsensor die Genauigkeit der Erfassung erhöhen. Über der zu erfassenden Zone kann hierzu ein Infrarotsender angebracht werden, der exakt die zu überwachende Zone ausleuchtet. Der in der Vorrichtung mögliche Infrarotempfänger empfängt das individuelle Signal des Infrarotsenders und kann damit entweder direkt eine Indikation für die Handhygiene auslösen oder beispielsweise den Mindestschrittzahlschwellwert verändern. Wenn z.B. in einer speziellen Situation Betten in einem sehr geringem Abstand zueinander stehen, so dass die Schritterkennung allein zur Detektion einer Indikation für die Handhygiene nicht ausreicht, dann kann die zusätzliche Einbeziehung eines Infrarotsignals in den Algorithmus für die Entscheidung, ob eine Handhygienemaßnahme erforderlich ist, einen Genauigkeitsvorteil bringen.

Funkbasierte Verfahren können hier keine vergleichbare Trennschärfe bei der Abtrennung von Zonen bieten. Weiterhin können funkbasierte Verfahren Probleme mit anderen Geräten verursachen.

Besonders bevorzugt kann eine tragbare Vorrichtung zur Verbesserung der Hygiene, mindestens eine Einheit zur Detektion einer Desinfektionsbehandlung, eine Erfassungseinheit, die auf Basis einer ununterbrochenen Schrittfolge mit einer bestimmten Mindestanzahl von Schritten einen Ortswechsel erkennt, der typischerweise mit einer Indikation zur Handhygiene verbunden ist, und eine Warneinheit, die mit der Detektionseinheit und der Erfassungseinheit in Wirkverbindung steht, deren Ergebnisse auswertet und auf dieser Basis ein Signal für den Benutzer zur Durchführung einer Handhygienemaßnahme auslöst, umfassen. Die Erfassungseinheit entspricht im Wesentlichen der zuvor dargelegten Schrittzähleinheit.

Weiterhin bevorzugt kann eine tragbare Vorrichtung zur Verbesserung der Hygiene, mindestens eine Einheit zur Detektion einer Desinfektionsbehandlung umfassen, durch die eine Desinfektionsbehandlung mit einem Gassensor und/oder durch Betätigung einer dafür vorgesehenen Taste an der tragbaren Vorrichtung durch den Träger der Vorrichtung und/oder durch Näherung an einen Desinfektionsmittelspender mittels Funktechnologie und/oder einer Betätigung eines Desinfektionsmittelspenders feststellbar ist, wobei die Betätigung des Desinfektionsmittelspenders beispielsweise durch Funktechnologie an die tragbare Vorrichtung übermittelt werden kann.

In einer weiteren besonderen Ausprägung der Erfindung umfasst die tragbare Vorrichtung der vorliegenden Erfindung weitere Sensoren oder zusätzliche Eingabeelemente. Dadurch kann beispielsweise der Träger einer Vorrichtung zusätzlich bestimmte Arbeitssituationen, z.B. Arbeit in der Intensivstation, die Durchführung einer aseptischen Tätigkeit oder einen Kontakt mit Körperflüssigkeiten anzeigen. In einer solchen Ausprägung kann eine lückenlose Erfassung aller Indikationen für eine Handhygiene nach WHO möglich sein. In einer weiteren besonderen Ausprägung der Erfindung kann durch Sensoren festgestellt werden, dass sich der Hygienepflichtige in einer Arbeitspause oder sonstigen Situation ohne Pflicht zur Einhaltung von Hygieneregeln befindet. Eine derartige Festlegung kann auch durch einen Träger der Vorrichtung über Eingabeelemente erfolgen.

Die Vorrichtung kann mit mindestens einem Datenspeicher ausgestattet werden, in welchem unter anderem Zeitdaten, Daten über die Anzahl der Handhygienebehandlungen und/oder Daten über die Handhygieneindikationen gemäß Schrittzahl oder anderen Detektionssystemen speicherbar sind, wobei der Datenspeicher vorzugsweise mit der Warneinheit und/oder der Einheit zur Detektion einer Desinfektionsbehandlung in Wirkverbindung steht. Vorzugsweise können alle relevanten Vorgänge, insbesondere alle durchgeführten Handhygienemaßnahmen und alle detektierten Indikationen für eine Handhygiene, versehen mit einem genauen Zeitstempel, in einen Datenspeicher geschrieben werden.

Die im Datenspeicher enthaltenen Daten können dann in einer weiteren vorteilhaften Ausprägung der Vorrichtung mittels einer Datenübertragungseinheit auf ein externes Computersystem übertragen werden.

Vorzugsweise ist die tragbare Vorrichtung mit mindestens einer Datenübertragungseinheit ausgestattet, die in Wirkverbindung mit dem Datenspeicher steht, so dass mit dem Datenspeicher enthaltene Daten auf ein externes Computersystem übertragbar sind.

Daraus können unter anderem statistische oder individuelle Informationen abgeleitet werden, die die Transparenz des erreichten Hygienestandes erhöhen und bei der Identifikation von weiteren Verbesserungsmöglichkeiten für Hygienemaßnahmen helfen.

Über eine geeignete Datenübertragungseinheit, die insbesondere eine Schnittstelle für bekannte Übertragungstechnologien aufweist, z.B. Bluetooth, WiFi oder kabelgebundene Standards wie z.B. USB, können die Daten aus dem Datenspeicher in ein Computersystem übertragen werden, um eine zentrale Datensammlung und statistische Auswertung ebenso wie eine individuelle Zurverfügungstellung der gesammelten Daten zu ermöglichen.

Dabei wird vorzugsweise darauf geachtet, dass nicht durch die Verwendung von Funktechnologien ggf. weitere Funksysteme in der anwendenden Organisation gestört werden.

Das vorzugsweise einsetzbare Computersystem dient der Sammlung der Speicherdaten aus den einzelnen erfindungsgemäßen Vorrichtungen in einer Organisationseinheit. In einer bevorzugten Ausprägung der Erfindung kann das Computersystem weiterhin der Aufladung der Akkus dienen, die vorzugsweise zur Stromversorgung der erfindungsgemäßen Vorrichtungen eingesetzt werden, indem diese nach Ende der Tätigkeit der Träger der Vorrichtungen zwecks Aufladung des Akkus und Übertragung der gespeicherten Daten mit dem Computersystem verbunden werden.

Eine erfindungsgemäße besondere Funktion des Computersystems besteht auch in einer möglichen Poolung der einzelnen tragbaren Vorrichtungen, die in einer Organisationseinheit eingesetzt werden. Dadurch wird eine Anonymisierung erreicht, ohne gleichzeitig auf einzelträgerbezogene Auswertungen verzichten zu müssen. Während also nicht zugeordnet werden kann, welche Person ein bestimmtes Desinfektionsverhalten an den Tag legt, kann sehr wohl festgestellt werden, dass an einem bestimmten Tag einer der Träger der Vorrichtung ein bestimmtes, z.B. unerwünschtes, Desinfektionsverhalten an den Tag gelegt hat. Die vorliegende Erfindung ermöglicht eine Abwägung der akzeptanzfördernden und ggf. datenschutzgerechten Wirkung von Anonymisierungsmaßnahmen mit den erweiterten Kontrollmöglichkeiten durch eine Personalisierung der erfindungsgemäßen Vorrichtungen.

Darüber hinaus kann den einzelnen Nutzern, auch im Fall einer grundsätzlichen Anonymität der Nutzung der tragbaren Vorrichtung durch Einrichtung eines gemeinsamen Pools von tragbaren Vorrichtungen, aus dem nicht individuell identifizierbare tragbare Vorrichtungen entnommen werden können und in den sie nach der Tragezeit (z.B. Schichtzeit) zurückgegeben werden können, dennoch die Möglichkeit für eine individuelle, zugriffgeschützte statistische Auswertung gegeben werden. Dies ist möglich, indem der Nutzer der tragbaren Vorrichtung und/oder dem System über eine geeignete Schnittstelle einen persönlichen und nur ihm bekannten Code mitteilt.

Diese Mitteilung kann über eine Funktechnologie oder über einen in der tragbaren Vorrichtung eventuell vorhandenen Infrarotempfänger erfolgen. Ferner kann durch Prüfsummen verifiziert werden, dass keine missbräuchliche Verwendung eines fremden Codes erfolgt. Der Datensatz von der Entnahme der mobilen Vorrichtung aus dem Pool bis zur Rückgabe der mobilen Vorrichtung in den Pool wird dann dem Nutzer zugeordnet, der sich durch seinen Code entsprechend identifiziert hat. Damit können individuelle Nutzungsstatistiken und Statistiken zum Desinfektionsverhalten erstellt werden, die nur dem Nutzer zugänglich sind, etwa durch Eingabe seines persönlichen, nur ihm bekannten Codes oder eines anderen Passwortes in einem EDV-System. Auf diese Weise können die Vorteile einer Anonymität in Bezug auf Datenschutz und Akzeptanz mit den Vorteilen der Möglichkeit der individuellen Einsichtnahme in die eigenen Daten verbunden werden.

Das Computersystem kann einzeln oder vernetzt eingesetzt werden. Insbesondere in größeren Einheiten kann eine Vernetzung, also ein Zusammenschluss mehrerer Computersysteme zweckmäßig sein, beispielsweise um eine statistische Kontrolle der einzelnen Organisationseinheiten zu ermöglichen. Ein Netzwerk, umfassend mindestens ein Computersystem, kann der Übertragung, Sammlung und Aggregation der Daten aus den verschiedenen Computersystemen dienen, die etwa in unterschiedlichen Einheiten einer größeren Organisation aufgestellt sind. Im Allgemeinen umfasst ein Netzwerk mindestens einen Server, der über mindestens einer Datenverbindung mit den jeweiligen Computersystemen verbunden ist.

Weiterhin können die Daten aus dem Netzwerk verschiedenen Nutzern zur Verfügung gestellt werden, etwa der Verwaltung der Organisation oder den einzelnen Trägern der erfindungsgemäßen Vorrichtungen. Die Daten können erfindungsgemäß statistisch aufbereitet, insbesondere in Zeitreihen, zur Verfügung gestellt werden, um auf dieser Basis Verbesserungsmaßnahmen einleiten zu können.

In einer möglichen Ausprägung des Systems können statistische Informationen über das Händedesinfektionsverhalten in verschiedenen Organisationseinheiten auf verschiedenen Endgeräten, z.B. auch PCs, Tablet-Computern, Mobilgeräten mit entsprechender Benutzerverwaltung und Zugangskontrolle angezeigt werden. Die angezeigten Informationen können beispielsweise die Anzahl der Desinfektionen pro Kalenderzeiteinheit oder die Anzahl der Desinfektionen je Stunde, in der die tragbare Vorrichtung getragen wurde oder die Anzahl der Desinfektionen je Desinfektionserinnerung durch die Warneinheit umfassen. Neben Zeitreihenanalysen sind auch Vergleiche der entsprechenden Werte für verschiedene Organisationseinheiten möglich.

In einer besonderen Ausprägung der Erfindung wird der mobile Einsatz der Vorrichtung dadurch erleichtert, dass die Vorrichtung besonders leicht gebaut wird. Gewichtstreiber der Vorrichtung nach dem bisherigen Stand der Technik ist insbesondere die Stromversorgung, z.B. in der Form eines Akkus. Der Stromverbrauch vor allem des Gassensors, insbesondere auch in der Ausprägung als Alkoholsensor, ist hoch, da das Messverfahren in handelsüblichen Sensoren die Nutzung eines Heizstroms beinhaltet. Das erfindungsgemäße Verfahren sieht in einer Ausprägung vor, dass der Stromverbrauch des Gassensors dadurch verringert wird, dass entgegen der Verwendung des Gassensors gemäß Spezifikation und bisherigem Stand der Technik die Spannungsversorgung auf ein Niveau unterhalb der gemäß Sensorspezifikation vorgeschriebenen Spannung geregelt wird. Dadurch wird zwar die Messgenauigkeit verringert, aber die Spannungsversorgung wird nur so weit reduziert, dass die für die Detektion der sehr stark konzentrierten Gaswolke bei der Durchführung einer Handhygienemaßnahme erforderliche Genauigkeit und Geschwindigkeit noch gegeben ist.

Ferner kann vorgesehen sein, dass die Einheit zur Detektion einer Desinfektionsbehandlung einen Halbleiter umfasst, wobei die Heizspannung für das Sensorelement nur kurzzeitig angelegt wird und das Signal des Gassensorelements bereits höchstens 250ms nach Heizbeginn gemessen wird.

In einer weiteren besonderen Ausprägung der Erfindung wird der Stromverbrauch des Gassensors verringert, indem entgegen der Sensorspezifikation nicht vorgeheizt und eingepegelt wird, sondern das Ausgangssignal des Sensors bereits in einem sehr kurzen zeitlichen Abstand nach dem Einschalten der Heizspannung - insbesondere höchstens 250ms nach dem Einschalten - gemessen wird. Dieses Ausgangssignal unterscheidet sich bei der im Zuge einer Handhygienemaßnahme gegebenen hohen Gaskonzentration hinreichend deutlich vom sonst üblichen Ausgangssignal, so dass eine eindeutige Messung und Unterscheidung möglich ist. Diese Kurzzeitmessungen müssen nur intervallweise - z.B. jede Sekunde - wiederholt werden, so dass eine Energieersparnis für einen Großteil der ansonsten durchgängig beheizten Zeit erzielt wird.

In einer weiteren besonderen Ausprägung der Erfindung wird eine Kombination der beiden oben angeführten Energiesparmaßnahmen, insbesondere Versorgungsspannungsreduktion und intervallweise Kurzzeitmessung, dergestalt eingesetzt, dass das Energieverbrauchsoptimum erreicht wird. Dabei führt die Reduktion der Heizspannung zu einem längeren erforderlichen Zeitraum, nach dem eine eindeutig diskriminierende Messung der kritischen Gaskonzentration in der intervallweisen Kurzzeitmessung möglich ist. Insgesamt führt aber eine Reduktion der Versorgungsspannung in Kombination mit der Stromverbrauchsreduktion durch intervallweise Kurzzeitmessung zu einem geringeren Stromverbrauch als die alleinige Verwendung eines dieser beiden Energiesparansätze.

Besonders bevorzugte Gassensoren basieren insbesondere auf Halbleitern, beispielsweise Zinndioxid (SnO₂). Diese Sensoren können beispielsweise unter der Bezeichnung MQ303A kommerziell erhalten werden. Im speziellen Fall der Verwendung des Alkoholsensors MQ303A kann bei einer Versorgungsspannung von 0,7V (statt spezifikationsgemäßen 0,9V) nach unter 250ms Aufheizzeit (statt mehreren Minuten oder gar Stunden) eine eindeutige Entscheidung über das Vorliegen einer Alkoholkonzentration oberhalb des Schwellwerts getroffen werden. Durch Verzicht auf die für die erfindungsgemäße Verwendung unnötige Genauigkeit wird der Stromverbrauch und damit auch das Akkugewicht (der Alkoholosensor ist der mit Abstand größte Verbraucher bei Verwendung typischer Bauteile) auf einen kleinen Bruchteil des Stromverbrauchs gesenkt, der bei der Verwendung des Alkoholsensors gemäß Spezifikation und Stand der Technikbesteht.

Ferner kann vorgesehen sein, dass zusätzlich die Heizspannung gegenüber dem spezifikationsgemäßen Maß reduziert wird. Das spezifikationsgemäße Maß ergibt sich hierbei aus den Angaben des Gassensorherstellers.

Das Verfahren kann in einer besonderen Ausprägung durch verfeinert werden, dass die Zeitdauer der Heizspannungsunterbrechung in Abhängigkeit von der in der Schrittzähleinheit gemessenen Schritthäufigkeit gewählt wird, wobei bei einer geringen Schritthäufigkeit die Zeitdauer der Spannungsunterbrechung größer ist als bei einer hohen Schritthäufigkeit.

Der geringe Stromverbrauch führt in dieser vorteilhaften Ausprägung der Erfindung dazu, dass mit der Vorrichtung während eines langen Zeitraums ohne erneute Aufladung des Energiespeichers permanent und automatisch das Vorliegen einer Handhygienemaßnahme gemessen werden kann und insofern eine gesonderte Aktivierung der Messung durch den Benutzer nicht erforderlich ist. Dadurch wird die Akzeptanz der Nutzung der Vorrichtung wesentlich gefördert.

In Bezug auf den oben beschriebenen, grundlegenden Ansatz 3 - Steigerung der Transparenz des individuellen Handhygieneverhaltens - erzielt die Erfindung in einer besonderen Ausprägung dadurch besondere Vorteile, dass sowohl die Detektion von erforderlichen Handhygienemaßnahmen, als auch die erfolgreiche Durchführung von Handhygienemaßnahmen, als auch die Signalgebung an den Hygienepflichtigen in einer einzigen, mobilen Vorrichtung integriert werden können, und somit Funkübertragungen und Aufwand durch eine Pluralität von Geräten vermieden werden können.

Ein besonderer Vorteil der Erfindung ist, dass diese mit einem sehr geringen Gewicht ausgeführt werden kann und somit leicht und komfortabel, ohne Beeinträchtigung der Bewegungsfreiheit als mobiles Gerät an der Arbeitskleidung der Hygienepflichtigen befestigt werden kann. Dadurch wird insbesondere auch ermöglicht, die Vorrichtung in Bauch oder Brusthöhe zu befestigen und somit eine besonders Zuverlässigkeit bei der automatischen Detektion von Handhygienemaßnahmen durch Messung der Gaskonzentration zu erreichen.

Ein weiterer Gegenstand der vorliegenden Erfindung ist ein System zur Verbesserung der Hygiene in medizinischen Einrichtungen, umfassend mindestens einen Spender, der dafür ausgelegt ist, ein Desinfektionsmittel an einen Nutzer auszugeben, und mindestens eine tragbare Vorrichtung gemäß der vorliegenden Erfindung.

Spender für Desinfektionsmittel sind in der Fachwelt weithin bekannt. Hierbei kann der Spender mit einem System ausgestattet sein, welches Daten mit der erfindungsgemäßen tragbaren Vorrichtung austauschen kann, um einen Desinfektionsvorgang zu detektieren. Gemäß einer bevorzugten Ausführungsform können Spender eingesetzt werden, die nicht mit einem spezifischen System zur Datenübertragung ausgestattet sind. In diesem Fall umfasst die Einheit zur Detektion einer Desinfektionsbehandlung der erfindungsgemäßen Vorrichtung mindestens einen Gassensor.

Gemäß einer weiteren Ausführungsform können auch Spender eingesetzt werden, die mit einem spezifischen System zur Datenübertragung ausgestattet sind. In diesem Fall kann die Einheit zur Detektion einer Desinfektionsbehandlung der erfindungsgemäßen Vorrichtung vorzugsweise mindestens eine weitere Einheit zum Austausch von Daten mit dem Desinfektionsmittelspender umfassen, so dass die Information über die Betätigung des Desinfektionsmittelspenders zur erfindungsgemäßen tragbaren Vorrichtung gelangen kann. Der Datenaustausch kann über Funkverbindungen erfolgen.

Gemäß einer weiteren Ausführungsform können auch Spender eingesetzt werden, die über einen Funksender (bevorzugt ein RfID-tag) oder Funkempfänger (bevorzugt ein RfID-Reader) verfügen. In diesem Fall muss das Gegenstück (bevorzugt ein RfID-Reader oder RfID-tag) in der erfindungsgemäßen tragbaren Vorrichtung vorhanden sein. Die Detektion einer Desinfektionsbehandlung erfolgt dann über die Auswertung der räumlichen Nähe zu einem Desinfektionsmittelspender über einen gewissen Mindestzeitraum. Dieses Verfahren zur Detektion einer Desinfektionsbehandlung kann mit weiteren Verfahren zur Erhöhung der Genauigkeit (z.B. Aktivierung des Funksenders oder Funkempfängers erst durch Betätigung des Hebels des Desinfektionsmittelspenders) kombiniert werden.

Die erfindungsgemäße tragbare Vorrichtung ist jeweils dadurch gekennzeichnet, dass die beliebige Einheit zur Detektion einer Desinfektionsbehandlung mit den weiteren erfindungsgemäßen Elementen kombiniert wird.

Weitere Ausgestaltungen des Systems zur Verbesserung der Hygiene ergeben sich aus der Beschreibung der erfindungsgemäßen Vorrichtung und des Computersystems, welches bevorzugt in einem Netzwerk eingesetzt wird.

So kann vorgesehen sein, dass die durch die tragbaren Vorrichtungen gesammelten Daten zentral erfassbar und statistisch auswertbar sind. Die gesammelten Daten können zentral aggregiert und ausgewertet werden, so dass daraus Rückschlüsse zur Verbesserung der Hygiene gezogen werden können.

Ferner kann vorgesehen sein, dass die aus der statistischen Auswertung der durch die Vorrichtungen gesammelten Daten zur Verbesserung der Hygiene herangezogen werden. Dies kann beispielsweise durch eine statistische Auswertung erfolgen. Diese statistischen Auswertungen können beispielsweise unter Berücksichtigung folgender Daten:
- Personaleinsatzstunden, insbesondere gemessen durch den Beschleunigungssensor
- Personaleinsatzstunden, gemessen durch die Zeit, in der sich die erfindungsgemäße tragbaren Vorrichtung außerhalb ihrer Basisstation zur Aufladung des Akkus befindet
- Anzahl der Personen, insbesondere Anzahl der Pflegekräfte und Ärzte
- Anzahl der Personen, insbesondere Patienten und deren Fallmix
- Kalendertag, Tageszeit
- Desinfektionshäufigkeit rechtzeitig vor ortswechselbezogenen Indikationen
- Desinfektionshäufigkeit nach Erinnerungen (Stufe 1, 2, 3)
- Zahl an verpassten Desinfektionen trotz Erinnerung
- in Verbindung mit Daten zu nosokomialen Infektionen
- etc., weitere Klinikdaten
erfolgen. Zur Auswertung können die zuvor dargestellten Datentypen einzeln oder in Kombination von mehreren verschiedenen Datenarten herangezogen werden. Hierdurch können Erfolge von Schulungsmaßnahmen überprüft werden. Weiterhin können Schulungsmaßnahmen an spezifische Erkenntnisse und Probleme angepasst werden. Weiterhin können ggf. spezielle Probleme in der Organisationseinheit, z.B. eine mangelnde Verfügbarkeit von Desinfektionsmittel vor der Auffüllung der Spender zu Wochenbeginn, anhand der Daten erkannt werden.

Ein weiterer Gegenstand der vorliegenden Erfindung ist ein Verfahren zur Verbesserung der Hygiene in Einrichtungen mit hohen Hygieneanforderungen, vorzugsweise medizinischen Einrichtungen, pharmazeutischen Unternehmen und/oder lebensmittelverarbeitenden Betrieben, umfassend den Einsatz einer tragbaren Vorrichtung gemäß der vorliegenden Erfindung. Besonders bevorzugt kann das Verfahren in medizinischen Einrichtungen umgesetzt werden.

Im Einzelnen kann dieses Verfahren dadurch gekennzeichnet sein, dass die Warneinheit nach einer vorgegebenen Zahl an unmittelbar hintereinander folgenden Schritten ein Signal auslöst, wobei vorzugsweise eine Umschaltung einer Kontrollanzeige zur Anzeige des Desinfektionszustandes erfolgt und/oder ein Vibrationsalarm ausgelöst wird. Die vorgegebene Zahl an unmittelbar hintereinander folgenden Schritten ist hierbei insbesondere von der Art und dem Aufbau der Einrichtung abhängig, deren Hygiene kontrolliert und/oder verbessert werden soll. Im Allgemeinen kann die vorgegebene Zahl an unmittelbar hintereinander folgenden Schritten im Bereich von 1 bis 50, vorzugsweise 2 bis 20 und besonders bevorzugt 3 bis 15 Schritten liegen. Diese Zahl kann gegebenenfalls durch einfache Versuche an ein Optimum angepasst werden, welches für eine spezifische Einrichtung gegeben ist. In Krankenhäusern liegt diese Zahl vorzugsweise im Bereich von 4 bis 12, insbesondere 5 bis 10 und besonders bevorzugt 6 bis 8 Schritten.

Vorzugsweise wird hierbei geprüft, ob unmittelbar vor oder nach Beginn der unmittelbar hintereinander folgenden Schritte eine Desinfektionsbehandlung erfolgt ist oder begonnen wurde. Im Allgemeinen ist der Desinfektionszustand eines Hygienepflichtigen auch nach mehr als der zuvor dargelegten Zahl an unmittelbar hintereinander folgenden Schritten gut, falls diese Schrittfolge nicht unterbrochen wurde. Demgemäß kann eine Umschaltung einer Kontrollanzeige zur Anzeige des Desinfektionszustandes und/oder ein Vibrationsalarm unterbleiben, falls unmittelbar vor oder nach Beginn der unmittelbar hintereinander folgenden Schritte eine Desinfektionsbehandlung erfolgt ist oder begonnen wurde.

Der Ausdruck "unmittelbar hintereinander folgenden Schritten" bezeichnet ein Bewegungsmuster, welches bei einer üblichen gleichförmigen Schrittfolge durch die tragbare Vorrichtung detektiert wird. Neben den Beschleunigungswerten sind hierbei insbesondere die zu diesen Beschleunigungswerten zugehörigen Zeitwerte von Interesse.

In einer vorteilhaften Ausprägung kann das Verfahren dadurch gekennzeichnet sein, dass durch die Warneinheit am Ende einer zeitlich gleichmäßigen, ununterbrochenen Schrittfolge, in der ein festgelegter Schwellwert für die Schrittanzahl überschritten wurde, ein Signal, vorzugsweise ein Vibrationsalarm ausgelöst wird.

Eine gleichmäßige, ununterbrochene Schrittfolge kann dabei dadurch detektiert werden, dass die Maxima der Beschleunigungswerte in der Vertikalbewegung, die der Schrittsensor detektiert, innerhalb einer bestimmten zeitlichen Abfolge liegen, also Maxima immer innerhalb eines Zeitraums von z.B. 0,4-1,2 Sekunden aufeinanderfolgen müssen. In einer besonders bevorzugten Ausführungsform wird der Zeitraum, in dem die Maxima der Beschleunigungswerte aufeinanderfolgen müssen, anhand des individuellen Bewegungsmusters des Trägers der tragbaren Vorrichtung bestimmt.

Das Signal, das am Ende einer zeitlich gleichmäßigen, ununterbrochenen Schrittfolge, in der ein festgelegter Schwellwert für die Schrittanzahl überschritten wurde, gegeben werden kann, kann allein und ausschließlich oder zusätzlich zu dem zeitlich früher möglichen Signal erfolgen, welches ggf. bereits beim Erreichen eines Schrittschwellwerts (beispielsweise nach 6 Schritten) auslöst wird. Vorzugsweise kann sich das Signal, das am Ende einer zeitlich gleichmäßigen, ununterbrochenen Schrittfolge, in der ein festgelegter Schwellwert für die Schrittanzahl überschritten wurde, gegeben werden kann, von dem Signal unterscheiden, welches beim Erreichen eines Schrittschwellwerts (beispielsweise nach 6 Schritten) erzeugt wird.

In einer weiteren vorteilhaften Ausprägung erfolgt nach einer Wartezeit, entweder nach Erreichen des Schrittanzahlschwellwerts oder nach Ende der zeitlich gleichmäßigen, ununterbrochenen Schrittfolge, in der ein festgelegter Schwellwert für die Schrittanzahl überschritten wurde, ein weiterer Vibrationsalarm, falls innerhalb dieser Wartezeit keine Desinfektionsbehandlung begonnen wurde und/oder keine weiteren Schritte detektiert wurden. Dieser Vibrationsalarm kann sich vorzugsweise von dem Vibrationsalarm nach Erreichen eines festgelegten Schwellwerts für die Schrittanzahl und/oder nach Erreichen eines Schrittschwellwerts (beispielsweise nach 6 Schritten) unterscheiden.

So kann der Träger der Vorrichtung mit zusätzlichem Nachdruck an eine erforderliche Handhygienemaßnahme erinnert werden. In einer für viele Einrichtungen besonders vorteilhaften Ausprägung liegt diese Wartezeit im Bereich von 2 bis 30 Sekunden, besonders bevorzugt 3 bis 15 Sekunden.

In einer weiteren vorteilhaften Ausprägung des Verfahrens wird zusätzlich die Dauer der Desinfektionsbehandlung gemessen. Dies kann wichtig sein, da die vorgeschriebene Dauer einer wirksamen Desinfektionsbehandlung typischerweise 30 Sekunden beträgt. In dem Verfahren kann daher zusätzlich erst nach einer vorgegebenen Dauer der Desinfektionsbehandlung eine Kontrollanzeige zur Anzeige des Desinfektionszustandes umgeschaltet werden. Die Dauer der Desinfektionsbehandlung kann beispielsweise über einen Gassensor leicht ermittelt werden, wobei die Konzentration an zu detektierendem Gas zu mehreren Zeitpunkten gemessen wird.

Bei dem Verfahren wird im Allgemeinen eine Schrittzahl von zeitlich relativ gleichmäßig aufeinanderfolgenden Schritte detektiert. Im Gegensatz zu üblichen Schrittzählern, die eine Gesamtzahl von Schritten ermitteln, kommt es für die Erfindung darauf an, dass ein handhygienepflichter Ortswechsel, in einer besonderen Ausprägung ein Verlassen oder Betreten einer Patientenumgebung, erfolgt. Dieser ist in der Praxis durch eine bestimmte Zahl von zeitlich gleichmäßig aufeinanderfolgenden Schritten gekennzeichnet, während einzelne Schritte während einer im wesentlichen stehenden Tätigkeit an einem bestimmten Ort oder Arbeitsplatz in der Praxis durch eine zeitlich unregelmäßige Schrittfolge gekennzeichnet sind. Ortswechsel von einem Arbeitsplatz zu einem anderen sind hingegen zumeist mit einer bestimmten Mindestanzahl von unmittelbar hintereinander folgenden Schritten verbunden, die die Vorrichtung erkennt. Die Vorrichtung erkennt den typischen Abstand zwischen zwei Schritten des Trägers der Vorrichtung und bestimmt, ob der jeweils nächste Schritt im Rahmen der üblichen Schwankungsbreite im erwarteten zeitlichen Abstand erfolgt. Nur wenn dies der Fall ist, wird der Schritt als ein Teil der aktuellen, ununterbrochenen Schrittfolge gezählt. Nur wenn eine ununterbrochene Schrittfolge die Mindestanzahl von Schritten - z.B. 6 Schritte - erreicht, wird dies als Indikator für das Vorliegen einer Handhygieneindikation gewertet. Eine nach weniger als der Mindestanzahl von Schritten unterbrochene Schrittfolge deutet darauf hin, dass der aktuelle Arbeitsplatz (in einer besonderen Ausprägung: die Patientenumgebung) nicht verlassen wurde.

Besonders wichtig für die besondere Ausprägung des Verfahrens in Wechselspiel mit einem Erfassungssystem für Handhygienemaßnahmen ist der Produktvorteil der Erfindung, dass die Detektion der Handhygiene mobil erfolgt. Die Indikation zu einer Handhygiene kann z.B. in relativ weiter Entfernung von einem Desinfektionsmittelspender auftreten. Falls der Träger der Vorrichtung dann erst noch zum Desinfektionsmittelspender laufen muss, erhöht dies in der Praxis deutlich die Wahrscheinlichkeit, dass eine ordnungsgemäße Handhygiene unterbleibt, z.B. aus Bequemlichkeit oder Zeitnot. Bei der Verwendung von mobilen Spendern von Handhygienemitteln und deren Detektion durch einen mobilen Gassensor an der tragbaren Vorrichtung kann dieser Nachteil vermieden werden und dennoch eine lückenlose Aufzeichnung des Handhygieneverhaltens erreicht werden.

Bei Verwendung der Vorrichtung in Verbindung mit einem Datenspeicher und einer Datenübertragungseinheit können in Einrichtungen mit mindestens einem Handhygienemittelspender die durch die Vorrichtung(en) gesammelten Daten zentral aggregiert und ausgewertet und daraus Rückschlüsse zur Verbesserung der Hygiene gezogen werden. Um Datenschutzaspekten zu entsprechen und die Akzeptanz zu erleichtern, können die tragbaren Vorrichtungen anonym ausgegeben werden. Insbesondere können sie periodisch, vorzugsweise täglich, vor Beginn einer Arbeitsschicht einem Gerätepool, etwa in einer Ladestation, entnommen werden und nach Beginn der Arbeitsschicht dort wieder abgelegt und aufgeladen werden. In dieser zentralen Station kann weiterhin die Datenübertragung an einen zentralen Computer erfolgen.

In einem derartigen System können aus den gewonnenen Daten Rückschlüsse zur Verbesserung der Hygiene gezogen werden. Gewonnene Daten können unter anderen die Handhygienehäufigkeiten pro (durch die Verwendung des Gerätepools anonymisiertem) Individuum umfassen. Die Handhygienehäufigkeit kann auch mit Personaleinsatzstunden, insbesondere gemessen durch den Beschleunigungssensor, Kalendertag, Tageszeit, oder ggf. weiteren externen Daten der Einrichtung, z.B. zur Anzahl der nosokomialen Infektionen oder zu bestimmten Interventionen und Maßnahmen der Einrichtung zur Steigerung der Häufigkeit der Handhygienemaßnahmen, in Beziehung gesetzt werden.

Auch die Anzahl der Indikationen für die Handhygiene und die Anzahl dieser Indikationen, vor oder nach denen eine Handhygiene tatsächlich erfolgte, oder erst nach Erinnerung erfolgte, kann ermittelt, gespeichert und übertragen werden, so dass auf dieser Basis weitere Maßnahmen zur Steigerung der Compliance zielgerichtet initiiert werden können.

Die erfindungsgemäße Vorrichtung sowie das erfindungsgemäße System zur Verbesserung der Hygiene eignen sich insbesondere zum Einsatz in medizinischen Einrichtungen, beispielsweise Krankenhäusern und Pflegeeinrichtungen. Weiterhin können die Vorrichtung und das System der vorliegenden Erfindung in Schlachtbetrieben, in Restaurants, beispielsweise Schnellrestaurants, und anderen Betrieben, in denen Lebensmittel oder Pharmazeutika hergestellt oder verarbeitet werden, eingesetzt werden.

Ein weiterer Gegenstand der vorliegenden Erfindung ist die Verwendung eines Systems zur Verbesserung der Hygiene zur statistischen Erfassung von Daten zur Verbesserung und/oder Aufrechterhaltung der Hygiene.

### Anwendungsbeispiele

Nachfolgend soll eine bevorzugte Ausführungsform der vorliegenden Erfindung anhand von zwei Figuren beispielhaft beschrieben werden, ohne dass hierdurch eine Begrenzung der Erfindung erfolgen soll. Es zeigen
- Figur 1: eine bevorzugte Ausführungsform einer tragbaren Vorrichtung gemäß der vorliegenden Erfindung und
- Figur 2: ein schematisches Ablaufdiagramm.

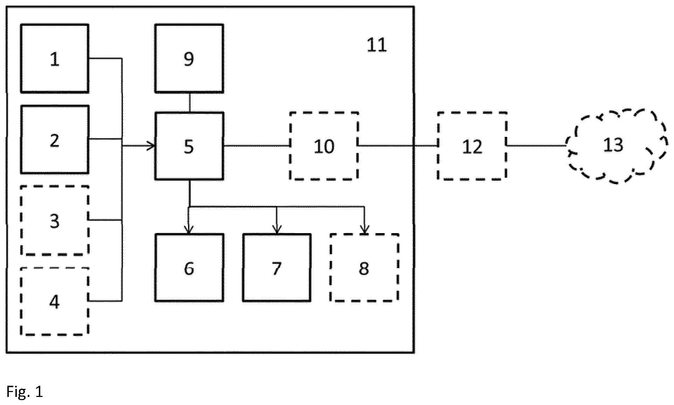

Figur 1 beschreibt eine bevorzugte Ausführungsform einer tragbaren Vorrichtung gemäß der vorliegenden Erfindung 11. Eine tragbare Vorrichtung 11 umfasst zumindest eine Warneinheit 5, vorliegend ein Mikrocontroller, der mit einer Schrittzähleinheit 1 in Wirkverbindung steht, vorliegend ein Beschleunigungsensor, dessen Daten über den in der Warneinheit 5 enthaltenen Mikrocontroller ausgewertet werden.

Vorliegend ist die Warneinheit 5 mit mindestens einer Schrittzähleinheit 1 über eine Datenverbindung derart verbunden, dass über die Schrittzähleinheit 1 erhaltene Daten in die Warneinheit 5 übertragbar sind, und die übertragenen Daten in der Warneinheit so verarbeitbar sind, dass ein Signal auslösbar ist.

Der von dem Mikrocontroller gesteuerte Signalgeber kann beispielsweise visuelle oder akustische Signale oder Vibrationen ausgegeben. Demgemäß kann eine tragbare Vorrichtung 11 fallweise zusätzlich eine LED-Leuchte 6 und/oder einen Alarmgeber 7 aufweisen, wobei besonders bevorzugte Ausführungsformen sowohl eine LED-Leuchte 6 und einen weiteren Alarmgeber 7 aufweisen. Die vorliegende Vorrichtung kann weiterhin mit einem zusätzlichen I/O-Element 8, beispielsweise einem Touchscreen ausgestattet werden.

Neben einer Warneinheit 5 und einer Schrittzähleinheit 1 umfasst eine erfindungsgemäße Vorrichtung mindestens eine Einheit zur Detektion einer Desinfektionsbehandlung 2, die in der in Figur 1 beschriebenen Ausführungsform als Gassensor ausgestaltet ist. Die vom Gassensor erhaltenen Daten können vorliegend über die Warneinheit 5 ausgewertet werden.

Vorzugsweise kann eine tragbare Vorrichtung 11 mindestens einen Infrarotsensor 3, über den besondere Bereiche detektiert werden können, so dass die Signalisierung an bestimmten Orten, den Bedürfnissen angepasst werden kann. Ferner kann die tragbare Vorrichtung ein weiteres Eingabeelement 4 aufweisen, über welches der Träger Einstellungen vornehmen kann. Der Infrarotsensor 3 und das Eingabeelement 4 sind mit der Warneinheit 5 verbunden.

Ferner kann die tragbare Vorrichtung 11 mindestens einen Datenspeicher 9 und mindestens eine Datenübertragungsschnittstelle 10 umfassen. In der in Figur 1 dargestellten Ausführungsform ist der Datenspeicher 9 an die Warneinheit angeschlossenen. Durch diese Ausgestaltung kann eine statistische Auswertung der vorgenommenen Desinfektionsbehandlung erfolgen.

Die Auswertung der Signale aus den Elementen 1-4 nach geeigneten Algorithmen, die Ausgabe von Signalen in die Elemente 6-8, die Speicherung der Ereignisse in Element 9 und die Ausgabe über Element 10 erfolgt vorliegend in Warneinheit 5 durch eine geeignete Datenverarbeitungseinheit, insbesondere in der Form eines Mikrocontrollers, die eine leichte Portabilität der Vorrichtung 11 ermöglicht.

Die Vorrichtung 11 kann vorzugsweise durch die Hygienepflichtigen an der Arbeitskleidung in Bauchhöhe getragen werden, um eine optimale Detektion von Handhygienemitteln durch den Gassensor 2 zu gewährleisten. Bei Handhygienemaßnahmen wird das Handhygienemittel während der Einwirkungszeit vielfach mit den Händen verrieben. Dazu werden die Hände im Allgemeinen mit typischerweise angewinkelten Armen in Bauchhöhe in geringem Abstand vor dem Körper bewegt. Dadurch entsteht eine sehr hohe lokale Gaskonzentration des Handdesinfektionsmittels, die bei geeigneter Parametrisierung der Konzentrationsschwelle für die Detektion einer Handhygienemaßnahme eine eindeutige Unterscheidung der Gaswolke durch eine Handhygienemaßnahme des Trägers der Vorrichtung von Hygienemaßnahmen in der Umgebung, etwa durch andere Personen, erlaubt, insbesondere auch durch eine mehrmalige Messung der Gaskonzentration.

Die tragbare Vorrichtung 11 kann vorzugsweise über eine Datenübertragungseinheit 10 mit einem Computersystem 12 verbunden werden. Das Computersystem 12 kann Teil eines Netzwerkes 13 sein, wobei die Datenverbindung durch übliche Standards, beispielsweise kabelgebunden über LAN oder durch Funktechnologien, beispielsweise WLAN erfolgen kann.

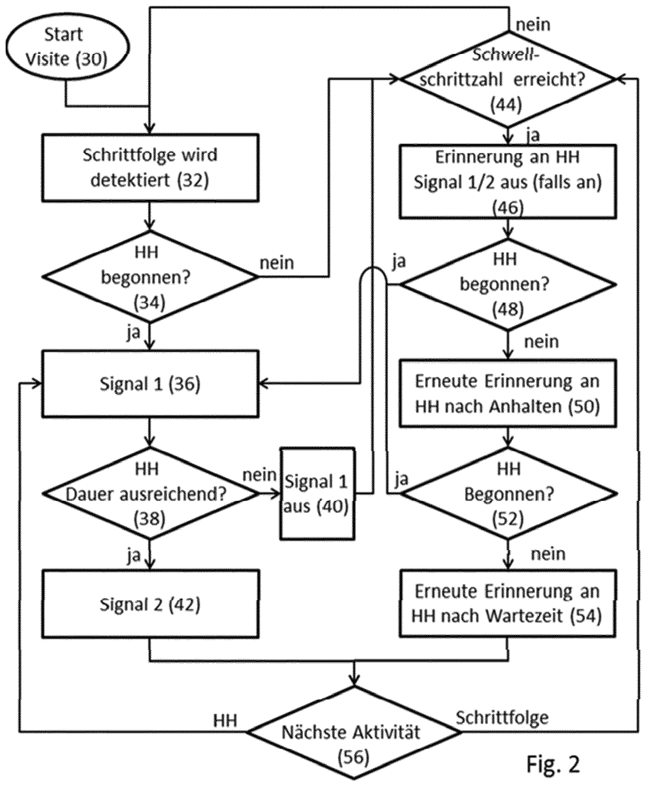

Figur 2 zeigt das Wechselspiel aus Schrittfolgen und erforderlichen Handdesinfektionsmaßnahmen, beispielsweise bei der Visite in einem Krankenhaus, und das erfindungsgemäße Verfahren dafür.

Eine Visite besteht aus einem Wechsel von typischerweise ununterbrochenen Schrittfolgen zum nächsten Patienten und Patientenbehandlungen mit typischerweise unterbrochenen Schrittfolgen.

Im erfindungsgemäßen Verfahren wird zu Beginn der Visite (30) in Verfahrensschritt (32) eine Schrittfolge detektiert, wobei die Zahl an zeitlich gleichmäßig aufeinanderfolgenden Schritten bestimmt wird.

In Entscheidungsschritt (34) wird geprüft, ob eine Handhygiene (HH) begonnen wurde. Diese Prüfung erfolgt vorzugsweise durch einen Gassensor.

Falls der Sensor einen Beginn einer Handhygiene (HH) feststellt, wird gemäß Verfahrensschritt (36) ein Signal 1, in einer bevorzugten Ausführung z.B. ein grünes Blinksignal, gegeben. In Entscheidungsschritt (38) wird auf eine ausreichende Desinfektionsmaßnahme geprüft.

Wird in Entscheidungsschritt (38) festgestellt, dass keine ausreichende Mindestzeit gegeben ist, wird das Signal 1 in Verfahrensschritt (40) ausgeschaltet und in Entscheidungsschritt (44) überwacht, ob die Schwellwertschrittzahl erreicht ist.

Wenn in einer weiteren bevorzugten Ausführung der Gassensor in Entscheidungsschritt (38) während einer Mindestzeit eine Mindest-Gaskonzentration misst, die auf eine Handhygienemaßnahme hindeutet, wird in Verfahrensschritt (42) ein Signal 2, in einer bevorzugten Ausführung grünes Dauerlicht, gegeben, unabhängig vom eventuellen Fortbestehen der Schrittfolge, und auch über deren Beendigung hinaus.

Signal 2 wird weiterhin während der aktuellen Schrittfolge und auch nach deren Beendigung gegeben, um zu indizieren, dass eine ausreichende Handhygiene erfolgt ist. Nach Beendigung der Schrittfolge, gekennzeichnet durch das Ausbleiben eines nächsten Schrittes im erwarteten zeitlichen Abstand, wird in Entscheidungsschritt (56) weitere Aktivität abgewartet, insbesondere ob entweder eine erneute Schrittfolge oder eine erneute Handhygienehandlung erfolgt. Eine erneute Handhygienehandlung führt zu einem Ablauf gemäß Verfahrensschritt (36), wohingegen eine erneut begonnene Schrittfolge zu einer Prüfung gemäß Entscheidungsschritt (44) führt.

Sofern in Entscheidungsschritt (34) festgestellt wird, dass keine Handhygienemaßnahme begonnen wurde, wird in Entscheidungsschritt (44) gemessen und überprüft, ob eine zuvor definierte Schwellwertschrittzahl, in einer bevorzugten Ausführung z.B. 6 gleichmäßig aufeinanderfolgende Schritte, erreicht ist.

Falls in Entscheidungsschritt (44) die Schwellwertschrittzahl nicht erreicht ist, wird weiterhin die aktuelle Schrittfolge beobachtet, wie dies in Verfahrensschritt (32) dargestellt ist. Gleichzeitig und/oder bei Ende der aktuellen Schrittfolge vor Erreichen der Schwellwertschrittzahl wird überprüft, ob eine Handhygienemaßnahme begonnen wurde (Entscheidungsschritt (34)). Falls ja, Verfahrensschritt (36), falls nein, wird in Entscheidungsschritt (44) weiterhin überprüft, ob eine ununterbrochene Schrittfolge die Schwellwertschrittzahl erreicht.

Sofern in Entscheidungsschritt (44) festgestellt wird, dass die Schwellwertschrittzahl erreicht ist, werden in Verfahrensschritt (46) etwaig noch angeschaltete Signale 1 und 2 ausgeschaltet und es wird per Signalgeber eine Handhygiene-Erinnerung, in einer bevorzugten Ausführung durch einen Vibrationsalarm, an den Träger der Vorrichtung ausgegeben. Falls danach in Entscheidungsschritt (48) der Beginn einer Handhygienemaßnahme detektiert wird, erfolgt das weitere Verfahren wie in Verfahrensschritt (36). Falls nicht, erfolgt eine erneute Erinnerung in Verfahrensschritt (50), in einer bevorzugten Ausführung durch einen Vibrationsalarm, nach Beendigung der aktuellen Schrittfolge. Wird danach in Entscheidungsschritt (52) der Beginn einer Handhygienemaßnahme detektiert, erfolgt das weitere Verfahren wie in Verfahrensschritt (36). Falls nicht, erfolgt in Verfahrensschritt (54) nach einer Wartezeit, in einer bevorzugten Ausführung z.B. nach 4-5 Sekunden, ein erneutes, vorzugsweise länger andauerndes Erinnerungssignal.

Danach wird in Entscheidungsschritt (56) auf weitere Aktivität, insbesondere entweder eine erneute Schrittfolge oder eine erneute Handhygienehandlung, gewartet.

Insgesamt ergibt sich hierdurch ein geschlossener Kreislauf an Entscheidungs- und Verfahrensschritten.

Im Folgenden ist als konkretes Beispiel für das Verfahren der typische Ablauf einer Visite in einem Krankenhaus geschildert, ohne dass durch diese Schilderung eine Begrenzung der Erfindung erfolgen soll. Nach gebäudeindividueller Analyse wird z.B. ein Standardwert von 6 Schritten als Schwellwert für eine Indikation für die Handhygiene in der Auswertungseinheit der Vorrichtung festgesetzt.
1. Der Träger der Vorrichtung (z.B. Ärztin oder Pflegekraft) betritt einen Raum. Dabei ist nach typischen Krankenhausregeln eine Handhygienemaßnahme erforderlich. Die Vorrichtung wird beim Betreten eines Raums typischerweise mehr als 6 Schritte detektieren. Entsprechend signalisiert das Gerät eine Indikation für die Handhygiene (z.B. durch Erlöschen einer grünen LED, die nach einer Handhygienemaßnahme angeschaltet wird, oder z.B. durch einen Vibrationsalarm für den Träger der Vorrichtung)
2. Bei einer erfolgreich durchgeführten Handhygienemaßnahme detektiert der Gassensor über eine gewissen Zeitraum eine chemische Substanz und schaltet danach ein Signal (z.B. eine grüne LED)
3. Während der Handhygienemaßnahme durchgeführte Schrittfolgen führen nicht zu einer erneuten Handhygieneindikation und werden somit von der Vorrichtung entsprechend behandelt.
4. Während der Einwirkungsdauer der Handhygienemaßnahme kommt der Träger der Vorrichtung beim Patienten an. Die Status-LED zeigt danach "grün" für eine erfolgreich durchgeführte Handhygiene. Innerhalb der Patientenumgebung bewegt sich der Träger der Vorrichtung typischerweise nicht mehrere Schritte gleichmäßig, sondern eher in Form einzelner Schritte, unterbrochen durch Pausen. Diese Einzelschritte werden nicht als Schrittfolge mit mindestens 6 Schritten erkannt. Insofern verändert sich der durch die Vorrichtung angezeigte Handhygienestatus zu Recht nicht: Es liegt objektiv keine erneute ortswechselbezogene Indikation für die Handhygiene vor.
5. Eine besonders vorteilhafte Ausprägung ist auch, dass ein ununterbrochener, durch regelmäßige Schrittfolge gekennzeichneter Gang, der bereits während der Dauer eine Handhygienemaßnahme begonnen wurde, nicht zu einer erneuten Handhygieneindikation führt, da die Hände dann am Zielort typischerweise ebenfalls noch hygienisch sind (das Öffnen einer Tür unterwegs führt zu einer erkennbaren Abweichung von der Regelmäßigkeit der Schrittfolge). Dadurch können am Zielort Verrichtungen vorgenommen werden, und die Indikator-LED zeigt zutreffend einen ausreichenden Hygienestatus an.
6. Wenn der Träger der Vorrichtung sich mehr als 6 Schritte innerhalb einer durchgängigen, ununterbrochenen Schrittfolge bewegt, dann hat dies in der Praxis fast immer eine Handhygieneindikation zur Folge. Beispiele sind der Gang zu einem Schrank im Patientenzimmer, der Gang zum Schreibtisch für eine Notiz oder der Gang zum Fenster. Indikation für eine Handhygiene nach WHO-Regeln, typischen Krankenhausregeln und Detektion einer Schrittfolge mit mehr als 6 Schritten decken sich in diesem Fall.
7. Nach Patientenkontakt und auch nach Verlassen einer Patientenumgebung erfolgt jeweils wieder eine Ortswechsel mit typischerweise mehr als 6 Schritten, so dass sich wiederum die Indikation für eine Handhygiene nach WHO-Regeln und nach typischen Krankenhausregeln sowie die Detektion einer Schrittfolge mit mehr als 6 Schritten decken.
8. Zusätzlich kann in einer besonderen Ausprägung des Verfahrens eine Erinnerung des Trägers sofort nach Ablauf von 6 ununterbrochenen Schritten eines Gangs eine Erinnerung erfolgen, dann erneut nach Abschluss des ununterbrochenen Gangs (z.B. nach 20 Schritten, sobald der Träger der Vorrichtung z.B. am nächsten Patientenbett angekommen ist), und einige Sekunden nach Abschluss des ununterbrochenen Gangs erneut zur nochmaligen Erinnerung an die erforderliche Handhygienemaßnahme, sofern nicht zwischenzeitlich der Beginn einer Handhygienemaßnahme durch den Gassensor detektiert wurde. So kann ausschließlich an tatsächlich erforderliche Handhygienemaßnahmen erinnert werden, an diese erforderlichen Maßnahmen aber besonders nachhaltig und falls erforderlich mehrfach.

## Patentansprüche

1. Tragbare Vorrichtung (11) zur Verbesserung der Hygiene, umfassend mindestens eine Warneinheit (5), die mit einer Einheit zur Detektion einer Desinfektionsbehandlung (2) derart zusammenwirkt, dass ein Signal auslösbar ist, **dadurch gekennzeichnet, dass** die tragbare Vorrichtung mindestens eine Schrittzähleinheit (1) aufweist und dass die Warneinheit mit der mindestens einen Schrittzähleinheit (1) in Wirkverbindung steht.

2. Tragbare Vorrichtung gemäß Anspruch 1, **dadurch gekennzeichnet, dass** die Schrittzähleinheit einen Beschleunigungssensor umfasst, der so ausgestaltet ist, dass über erfasste Daten mit geeigneter Software ein Bewegungsmuster erkennbar ist, welches einer vorgegebenen Zahl an unmittelbar hintereinander folgenden Schritten und/oder einer Schrittzahl von zeitlich relativ gleichmäßig aufeinanderfolgenden Schritte entspricht.

3. Vorrichtung gemäß Anspruch 1 oder Anspruch 2, **dadurch gekennzeichnet, dass** die Einheit zur Detektion einer Desinfektionsbehandlung mindestens einen Gassensor aufweist, der vorzugsweise gegenüber Alkohol, besonders bevorzugt Ethanol und/oder Propanol, empfindlich ist.

4. Vorrichtung gemäß mindestens einem der vorhergehenden Ansprüche, **dadurch gekennzeichnet, dass** die Warneinheit eine Kontrollanzeige zur Anzeige des Desinfektionszustandes umfasst.

5. Vorrichtung gemäß mindestens einem der vorhergehenden Ansprüche, **dadurch gekennzeichnet, dass** die Warneinheit eine Einheit zur Erzeugung eines Vibrationsalarms umfasst.

6. Vorrichtung gemäß mindestens einem der vorhergehenden Ansprüche, **dadurch gekennzeichnet, dass** die tragbare Vorrichtung mindestens einen IR-Sensor umfasst, der in Wirkverbindung mit der Warneinheit steht.

7. Vorrichtung gemäß mindestens einem der vorhergehenden Ansprüche, **dadurch gekennzeichnet, dass** die tragbare Vorrichtung mindestens einen Datenspeicher umfasst, in welchem Zeitdaten, Handhygienemaßnahmen und/oder erkannte Handhygieneindikationen speicherbar sind, wobei der Datenspeicher vorzugsweise mit der Warneinheit und/oder der Einheit zur Detektion einer Desinfektionsbehandlung in Wirkverbindung steht.

8. Verfahren zur Verbesserung der Hygiene in Einrichtung mit hohen Hygieneanforderungen, vorzugsweise medizinischen Einrichtungen, pharmazeutischen Unternehmen und/oder lebensmittelverarbeitenden Betrieben, umfassend den Einsatz einer tragbaren Vorrichtung gemäß mindestens einem der Ansprüche 1 bis 7.

9. Verfahren gemäß Anspruch 8, **dadurch gekennzeichnet, dass** die Warneinheit nach einer vorgegebenen Zahl an unmittelbar hintereinander folgenden Schritten ein Signal auslöst, wobei vorzugsweise eine Umschaltung einer Kontrollanzeige zur Anzeige des Desinfektionszustandes erfolgt und/oder ein Vibrationsalarm ausgelöst wird.

10. Verfahren gemäß Anspruch 8 oder 9, **dadurch gekennzeichnet, dass** die Warneinheit am Ende einer zeitlich gleichmäßigen, ununterbrochenen Schrittfolge, in der ein festgelegter Schwellwert für die Schrittanzahl überschritten wurde, ein Signal auslöst wird, wobei vorzugsweise ein Vibrationsalarm ausgelöst wird.

11. Verfahren gemäß Anspruch 10, **dadurch gekennzeichnet, dass** nach einer Wartezeit ein weiterer Vibrationsalarm erfolgt, falls innerhalb dieser Wartezeit keine Desinfektionsbehandlung erfolgt ist und/oder keine weiteren Schritte detektiert wurden.

12. Verfahren gemäß mindestens einem der Ansprüche 9 bis 11, **dadurch gekennzeichnet, dass** die Dauer der Desinfektionsbehandlung gemessen wird.

13. System zur Verbesserung der Hygiene in medizinischen Einrichtungen, umfassend mindestens einen Spender, der dafür ausgelegt ist, ein Desinfektionsmittel an einen Nutzer auszugeben, und mindestens eine tragbare Vorrichtung gemäß mindestens einem der Ansprüche 1 bis 7.

14. System gemäß Anspruch 13, **dadurch gekennzeichnet, dass** die durch die tragbare Vorrichtung gesammelten Daten zentral erfassbar und statistisch auswertbar sind.

15. Verwendung eines Systems gemäß Anspruch 13 oder 14 zur statistischen Erfassung von Daten zur Verbesserung und/oder Aufrechterhaltung der Hygiene.

## Claims

1. A wearable device (11) for improving hygiene, comprising at least one warning unit (5) that cooperates with a unit for detecting a disinfection treatment (2) in such a way that a signal is triggerable, **characterized in that** the wearable device has at least one step counter unit (1), and the warning unit is in operative connection with the at least one step counter unit (1).

2. The wearable device according to Claim 1, **characterized in that** the step counter unit includes an acceleration sensor that is designed in such a way that a movement pattern is recognizable via detected data, using suitable software, the movement pattern corresponding to a predefined number of steps taken in direct succession and/or to a number of steps in relatively uniform temporal succession.

3. The device according to Claim 1 or Claim 2, **characterized in that** the unit for detecting a disinfection treatment has at least one gas sensor that is preferably sensitive to alcohol, particularly preferably ethanol and/or propanol.

4. The device according to at least one of the preceding claims, **characterized in that** the warning unit includes a control display for displaying the disinfection status.

5. The device according to at least one of the preceding claims, **characterized in that** the warning unit includes a unit for generating a vibrating alarm.

6. The device according to at least one of the preceding claims, **characterized in that** the wearable device includes at least one IR sensor that is in operative connection with the warning unit.

7. The device according to at least one of the preceding claims, **characterized in that** the wearable device includes at least one data memory in which time data, hand hygiene measures, and/or recognized hand hygiene indications are storable, wherein the data memory is preferably in operative connection with the warning unit and/or the unit for detecting a disinfection treatment.

8. A method for improving hygiene in a facility having strict hygiene requirements, preferably medical facilities, pharmaceutical companies, and/or food processing areas, comprising the use of a wearable device according to at least one of Claims 1 to 7.

9. The method according to Claim 8, **characterized in that** the warning unit triggers a signal after a predefined number of steps taken in direct succession, wherein switching over to a control display for displaying the disinfection status preferably takes place and/or a vibrating alarm is triggered.

10. The method according to Claim 8 or 9, **characterized in that** the warning unit triggers a signal at the end of a temporally uniform, uninterrupted sequence of steps in which a set threshold value for the number of steps has been exceeded, wherein a vibrating alarm is preferably triggered.

11. The method according to Claim 10, **characterized in that** after a waiting period, a further vibrating alarm occurs if, within this waiting period, no disinfection treatment has taken place and/or no further steps have been detected.

12. The method according to at least one of Claims 9 to 11, **characterized in that** the duration of the disinfection treatment is measured.

13. A system for improving hygiene in medical facilities, including at least one dispenser that is designed to dispense a disinfectant to a user, and at least one wearable device according to at least one of Claims 1 to 7.

14. The system according to Claim 13, **characterized in that** the data collected by the wearable device are centrally detectable and statistically evaluatable.

15. Use of a system according to Claim 13 or 14 for statistically recording data for improving and/or maintaining hygiene.

## Revendications

1. Dispositif portable (11) permettant d'améliorer l'hygiène, comprenant au moins une unité d'avertissement (5) qui coopère avec une unité de détection de traitement de désinfection (2) de telle sorte qu'un signal peut être déclenché, **caractérisé en ce que** le dispositif portable présente au moins une unité de comptage de pas (1) et **en ce que** l'unité d'avertissement est en liaison active avec ladite au moins une unité de comptage de pas (1).

2. Dispositif portable selon la revendication 1, **caractérisé en ce que** l'unité de comptage de pas comprend un capteur d'accélération qui est conçu de telle sorte qu'à l'aide de données collectées et d'un logiciel approprié, il est possible de reconnaître un schéma de mouvement lequel correspond à un nombre prédéterminé de pas se succédant immédiatement les uns aux autres et/ou à un nombre de pas se succédant les uns aux autres de manière relativement régulière dans le temps.

3. Dispositif selon la revendication 1 ou 2, **caractérisé en ce que** l'unité de détection de traitement de désinfection présente au moins un capteur de gaz qui est préférentiellement sensible à l'alcool, encore plus préférentiellement à l'éthanol et/ou au propanol.

4. Dispositif selon au moins l'une des revendications précédentes, **caractérisé en ce que** l'unité d'avertissement comprend un indicateur de contrôle servant à indiquer l'état de désinfection.

5. Dispositif selon au moins l'une des revendications précédentes, **caractérisé en ce que** l'unité d'avertissement comprend une unité servant à produire une alarme vibrante.

6. Dispositif selon au moins l'une des revendications précédentes, **caractérisé en ce que** le dispositif portable comprend au moins un capteur infrarouge qui est en liaison active avec l'unité d'avertissement.

7. Dispositif selon au moins l'une des revendications précédentes, **caractérisé en ce que** le dispositif portable comprend au moins une mémoire de données dans laquelle peuvent être enregistrées des données temporelles, des mesures d'hygiène manuelles et/ou des indications d'hygiène manuelles détectées, cette mémoire de données étant préférentiellement en liaison active avec l'unité d'avertissement et/ou l'unité de détection d'un traitement de désinfection.

8. Procédé permettant d'améliorer l'hygiène dans des établissements devant satisfaire à de hautes exigences en matière d'hygiène, préférentiellement dans des établissements médicaux, des entreprises pharmaceutiques et/ou des entreprises de transformation agro-alimentaires, comprenant l'utilisation d'un dispositif portable selon au moins l'une des revendications 1 à 7.

9. Procédé selon la revendication 8, **caractérisé en ce que** l'unité d'avertissement déclenche un signal après un nombre prédéfini de pas se succédant immédiatement les uns aux autres, un indicateur de contrôle servant à indiquer l'état de désinfection étant préférentiellement commuté et/ou une alarme vibrante déclenchée.

10. Procédé selon la revendication 8 ou 9, **caractérisé en ce que** l'unité d'avertissement déclenche un signal au bout d'une séquence de pas ininterrompue et régulière dans le temps, dans laquelle séquence une valeur seuil prédéfinie pour le nombre de pas est dépassée, l'alarme déclenchée consistant préférentiellement en une alarme vibrante.

11. Procédé selon la revendication 10, **caractérisé en ce qu'**après temps d'attente, une nouvelle alarme vibrante est déclenchée si, pendant ce temps d'attente, il n'a été effectué aucun traitement de désinfection et/ou détecté aucune autre mesure.

12. Procédé selon au moins l'une des revendications 9 à 11, **caractérisé en ce qu'**est mesurée la durée du traitement de désinfection.

13. Système permettant d'améliorer l'hygiène dans des établissements médicaux, comprenant au moins un distributeur, qui est conçu pour distribuer un produit de désinfection à un utilisateur, et au moins un dispositif portable selon au moins l'une des revendications 1 à 7.

14. Système selon la revendication 13, **caractérisé en ce que** les données collectées par le dispositif portable peuvent être saisies de manière centralisée et exploitées statistiquement.

15. Utilisation d'un système selon la revendication 13 ou 14 permettant la saisie statistique de données pour l'amélioration et/ou le maintien de l'hygiène.
